# EUROPEAN PATENT APPLICATION

(11) **EP 4 332 965 A1**
(43) Date of publication of application: **06.03.2024**
(21) Application number: 22193185.0
(22) Date of filing: 31.08.2022
(51) Int. Cl.: G10L 25/66, A61B 5/00, G10L 25/15

(54) **SYSTEM AND METHOD CONFIGURED FOR ANALYSING ACOUSTIC PARAMETERS OF SPEECH TO DETECT, DIAGNOSE, PREDICT AND/OR MONITOR PROGRESSION OF A CONDITION, DISORDER OR DISEASE**

(71) Applicant: Beats Medical Limited, Dublin 18 (IE)
(72) Inventor: CLANCY, CIARA LOURDA, Dublin (IE); PEREIRA CALDEIRA, VÁLTER JOSÉ, Dublin (IE); CALDEIRA PEREIRA, ANDRÉ, Dublin (IE)
(74) Representative: White, Jonathan Patrick

(57) **Abstract**

The present invention relates to a system and method configured for analysing acoustic parameters of speech to detect, diagnose, predict and/or monitor progression of a condition, disorder, or disease, and more particularly, any of paediatric and adult neurological and central nervous system conditions including but not limited to low back pain, multiple sclerosis, stroke, seizures, Alzheimer's disease, Parkinson's disease, dementia, motor neuron disease, muscular atrophy, acquired brain injury, cancers involving neurological deficits, paediatric developmental conditions and rare genetic disorders such as spinal muscular atrophy. The system and method extracts a first formant data set from words spoken by an individual and uses these to classify the vowels in the words on a first computing device, such as a mobile smart phone equipped with a microphone into which an individual speaks. The system stores at least some of these frequencies for the vowel formants in a second formant data set as a recorded file and provides the second formant data set as input to acoustic metrics to generate score data from which an assessment is made to determine the articulation level of the vowels in the words spoken by the individual, allowing for detection, diagnosis, prediction and/or monitoring progression of the condition, disorder, or disease.

## Description

The present invention relates to a system and method configured for analysing acoustic parameters of speech to detect, diagnose, predict and/or monitor progression of a condition, disorder, or disease, and more particularly, any of paediatric and adult neurological and central nervous system conditions including but not limited to low back pain, multiple sclerosis, stroke, seizures, Alzheimer's disease, Parkinson's disease, dementia, motor neuron disease, muscular atrophy, acquired brain injury, cancers involving neurological deficits, paediatric developmental conditions and rare genetic disorders such as spinal muscular atrophy.

Speech signals contain measurable acoustic parameters that are related to speech production, and by analysing the acoustics of speech, aspects related to motor speech functions may be deduced.

Moreover, acoustic analysis of human speech, and in particular, measuring vowel articulation impairment in individuals, may assist in detecting the presence, severity, and characteristics of motor speech disorders that are associated with conditions or diseases, such as Parkinson's or Alzheimer's disease, and/or the above paediatric and adult neurological and central nervous system conditions. By analysing the level of vowel articulation impairment of an individual's speech health professionals may also monitor deterioration or improvement in speech due to progression, recovery, or treatment effects related to the disease.

Formants, also known as harmonics, are frequency domain features of speech and are a concentration of acoustic energy around a particular frequency in a speech signal or wave. The formants, F1 (Hz) and F2 (Hz), and when required, F3 (Hz), are typically considered to be the frequencies of the acoustic parameters associated with the perception and production of vowels that are present in words articulated by an individual.

As the range of articulatory movements is often impaired (due to vocal tract constriction issues) in individuals with such conditions and/or diseases the ability for such individuals to articulate vowels contained in a word is also often impacted. By measuring the frequency of the F1,F2 and optionally, F3 vowel formants for an individual with such conditions and/or diseases and comparing these with the frequencies of the F1 and F2 formants for an individual with normal speech, the level of this impairment may be measured, and inferences drawn, as to the severity and treatment progression of the individual with such conditions and/or diseases.

The frequencies of such formants may be extracted from a speech wave and measured in a variety of ways, such as by using well known computer software packages for speech analysis in phonetics, such as PRAAT.

Speech and vowel articulation impairment and are thus well-known digital biomarkers for such conditions and/or diseases.

However, systems and methods configured for such acoustic analysis of human speech suffer from drawbacks including that they are required to process saved and therefore stored audio files encoding human speech to be analysed post hoc, which invariably drains memory resources, presents data privacy issues, and disallows feedback during the exercise. Additionally, such systems and methods lack near real time delivery of results.

It is a therefore an object of the present invention to provide a system and method that goes at least some way toward overcoming the above problems and/or which will provide the public and/or industry with a useful alternative.

Further aspects of the present invention will become apparent from the ensuing description which is given by way of example only.

According to the invention, there is provided a system configured for analysing acoustic parameters of speech to detect, diagnose, predict and/or monitor progression of a condition, disorder, or disease, the system comprising a first computing device configured with means for receiving an audio stream containing speech data encoding at least one word spoken by an individual, the first computing device is configured with:
means to covert the audio stream into a sound signal,
means to extract from the sound signal a first formant data set comprising formant frequencies associated with letters in the at least one word as the audio stream is being received,
means to determine from the first formant data set formant frequencies that are associated with one or more vowel letters in the word,
means to determine the specific vowel letter or vowel letters from the determined vowel letter formant frequencies,
means for recording a second formant data set comprising at least some of the determined vowel letter formant frequencies for the specific vowel letter or vowel letters,
whereby the system further comprises, executing on the first computing device and/or a second computing device coupled to the first computing device by a network, means to generate score data by applying one or more predetermined acoustic metrics to the recorded second formant data set, in which the score data is used to determine a level of articulation of the vowel letter or vowel letters in the at least one word spoken by the individual, and
means to store the score data as an output file.

The present invention is directed to a system and method that analyses vowel delivery in speech as it pertains to clinical presentation of a disorder, condition or disease in individuals suffering from any of paediatric and adult neurological and central nervous system conditions including but not limited to low back pain, multiple sclerosis, stroke, seizures, Alzheimer's disease, Parkinson's disease, dementia, motor neuron disease, muscular atrophy, acquired brain injury, cancers involving neurological deficits, paediatric developmental conditions and rare genetic disorders such as spinal muscular atrophy.

The invention provides a computer implemented system and method which determines whether an individual articulates vowels in a spoken word to detect the presence of the disease and monitor deterioration or improvement in speech due to progression, recovery, or treatment effects related to the disease.

The invention may be used to predict, diagnose and/or determine progression of disease.

The system extracts a first formant data set from words spoken by an individual and uses these to classify the vowels in the words on a first computing device, such as a mobile smart phone equipped with a microphone into which an individual speaks. The system stores at least some of these frequencies for the vowel formants in a second formant data set as a recorded file and provides the second formant data set as input to acoustic metrics to generate score data from which an assessment is made to determine the articulation level of the vowels in the words spoken by the individual, allowing for detection, diagnosis, prediction and/or monitoring progression of the condition, disorder, or disease.

For example, if the individual says the word "apple" into a microphone of the first computing device, the system is configured on the first computing device, to extract the vowel formants that are present in the word as spoken by the individual, record at least some of these as a second formant data set, and use the second formant data set as input to acoustic metrics to assess whether the individual has articulated the vowels "a" and "e" in the word "apple", which will service a purpose of understanding if the individual is saying the important vowel parts of the word.

Score data by way of a report on the level of articulation is generated which can allow for detection, improvements and assessing disease states. The score data may be generated on the first computing device and/or a second computing device coupled to the first computing device by a network

The invention is thus configured to classify vowels in words spoken by an individual based on formant frequencies in near real time and without having to record files containing the original speech of the individual since only a file containing required vowel formant frequency data is recorded. This arrangement significantly reduces memory requirements, lag in delivering results and addresses data privacy issues since it is only the vowel formant frequencies data extracted from an audio stream of an individual speech that are recorded.

Preferably, the formant frequencies in the first formant data set comprise at least formant frequencies F1 (Hz) and F2 (Hz).

Alternatively, the formant frequencies in the first formant data set comprise at least formant frequencies F1 (Hz), F2 (Hz) and F3 (Hz).

Thus, formant frequencies F1 (Hz), F2 (Hz) and optionally, F3 (Hz), are used to classify vowels in the word or words spoken by the individual.

Preferably, the formant frequencies in the second formant data set comprise formant frequencies F1 (Hz) and F2 (Hz).

Thus, formant frequencies F1 (Hz) and F2 (Hz) are used as input to the acoustic metrics to determine a level of articulation of the vowels in the word or words spoken by the individual.

Preferably, the first computing device is a mobile computing device, such as a mobile smart phone, having mobile telephone and computing functionality.

Preferably, the means for extracting the first formant data set comprises:
speech analyser means configured with means for converting the sound signal from a time domain signal to a frequency domain signal, such as by using a fast Fourier transform (FFT) algorithm, and
linear predictive coding means comprising means for applying an autocorrelation algorithm to estimate the dominating frequency in the frequency domain signal, and means for applying a Levinson-Durbin algorithm to estimate linear prediction parameters for the estimated dominating frequency to compress the frequency domain signal and identify signal peaks in the compressed frequency domain signal, and
means for decompressing the compressed frequency domain signal, and
means for extracting the identified signal peaks from the decompressed frequency domain signal,
whereby the extracted signal peaks correspond to the formant frequencies in the first formant data set.

Preferably, the means to determine from the first formant data set formant frequencies that are associated with the one or more vowel letters and to determine the specific vowel letter comprises means for applying a Mahalanobis distance algorithm to the extracted formant frequencies at the first computing device.

Preferably, the predetermined metrics comprise one or more of: a formant centralisation ratio (FCR) algorithm, a vowel space algorithm (VSA) and a vowel articulation index (VAI) algorithm.

According to a further aspect of the invention, there is provided a method configured for analysing acoustic parameters of speech to detect, diagnose, predict and/or monitor progression of a condition, disorder, or disease, the method comprising using a first computing device configured with means for receiving an audio stream containing speech data encoding at least one word spoken by an individual, the first computing device is configured to perform steps of:
converting the audio stream into a sound signal,
extracting from the sound signal a first formant data set comprising formant frequencies associated with the letters in the at least one word as the audio stream is being received,
determining from the first formant data set formant frequencies that are associated with one or more vowel letters in the word,
determining the specific vowel letter or vowel letters from the determined vowel letter formant frequencies,
recording a second formant data set comprising at least some of the determined vowel letter formant frequencies for the specific vowel letter or vowel letters,
whereby the method further comprises a step of, on the first computing device and/or a second computing device coupled to the first computing device by a network, generating score data by applying one or more predetermined acoustic metrics to the recorded second formant data set, in which the score data is used to determine a level of articulation of the vowel letter or vowel letters in the at least one word spoken by the individual, and
storing the score data as an output file.

Preferably, the formant frequencies in the first formant data set comprise at least formant frequencies F1 (Hz) and F2 (Hz).

Alternatively, the formant frequencies in the first formant data set comprise at least formant frequencies F1 (Hz), F2 (Hz) and F3 (Hz).

Thus, formant frequencies F1 (Hz), F2 (Hz) and optionally, F3 (Hz), are used to classify vowels in the word or words spoken by the individual.

Preferably, the formant frequencies in the second formant data set comprise formant frequencies F1 (Hz) and F2 (Hz).

Thus, formant frequencies F1 (Hz) and F2 (Hz) are used as input to the acoustic metrics to determine a level of articulation of the vowels in the word or words spoken by the individual.

Preferably, the first computing device is a mobile computing device, such as a mobile smart phone, having mobile telephone and computing functionality.

Preferably, the step of extracting first formant data set comprises:
converting the sound signal from a time domain signal to a frequency domain signal, such as by using a fast Fourier transform (FFT) algorithm, and
applying an autocorrelation algorithm to estimate the dominating frequency in the frequency domain signal, and applying a Levinson-Durbin algorithm to estimate linear prediction parameters for the estimated dominating frequency to compress the frequency domain signal and identify signal peaks in the compressed frequency domain signal, and
decompressing the compressed frequency domain signal, and
extracting the identified signal peaks from the decompressed frequency domain signal,
whereby the extracted signal peaks correspond to the formant frequencies in the first formant data set.

Preferably, the method comprises applying a Mahalanobis distance algorithm to the extracted formant frequencies at the first computing device to determine from the first formant data set formant frequencies that are associated with the one or more vowel letters and to determine the specific vowel letter.

Preferably, the predetermined metrics comprise one or more of: a formant centralisation ratio (FCR) algorithm, a vowel space algorithm (VSA) and a vowel articulation index (VAI) algorithm.

The invention will be more clearly understood from the following description of some embodiments thereof, given by way of example only, with reference to the accompanying drawings in which:
Figure 1 is a flow diagram showing processing steps in a method performed by a system configured according to the invention,
Figure 2 is a flow diagram showing processing steps performed to extract formant data from a sound signal according to the invention,
Figure 3 is a flow diagram showing processing steps performed to determine and classify vowels in the formant data extracted at Figure 2,
Figure 4 is a graph of cluster data showing formant frequency F2 (Hz) against formant frequency F1 (Hz) used for classifying vowel letters, and
Figure 5 is a flow diagram showing processing steps to determine using acoustic metrics whether an individual has articulated vowel letters in a word.

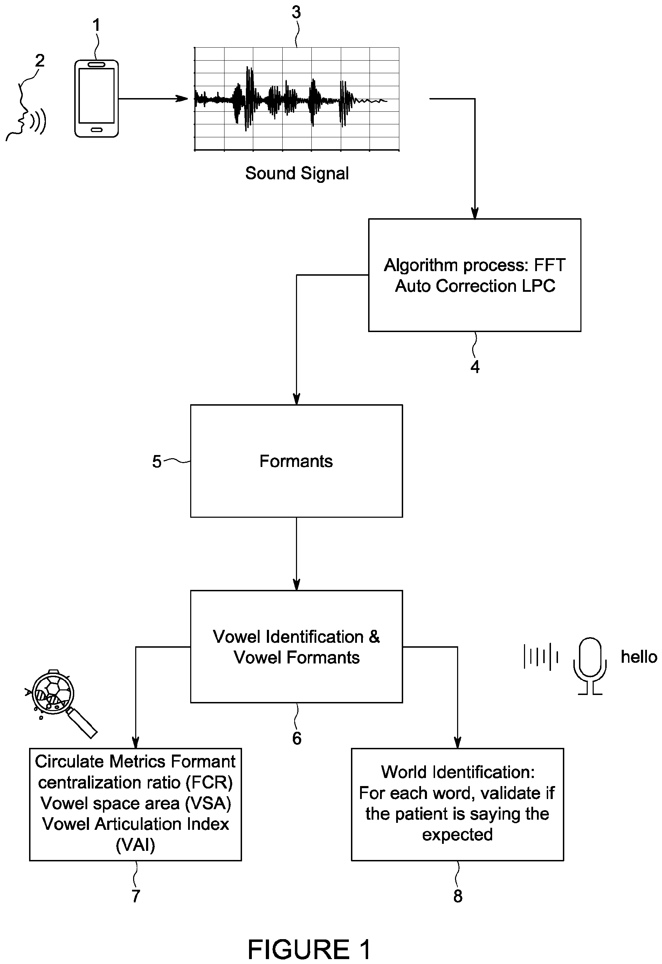

Referring to the drawings, and initially to Figure 1, there is provided a method configured for analysing acoustic parameters of speech to detect, diagnose, predict and/or monitor progression of a condition, disorder, or disease, including any of paediatric and adult neurological and central nervous system conditions including but not limited to low back pain, multiple sclerosis, stroke, seizures, Alzheimer's disease, Parkinson's disease, dementia, motor neuron disease, muscular atrophy, acquired brain injury, cancers involving neurological deficits, paediatric developmental conditions and rare genetic disorders such as spinal muscular atrophy.

The method comprises using a first computing device 1 configured with means for receiving an audio stream, such as a microphone, containing speech data encoding at least one word spoken by an individual 2.

The first computing device 1 is provided as a mobile smart phone configured to perform a step of converting the audio stream received into a sound signal 3.

From the sound signal 3 obtained a formant data extraction process 4 is performed to extract a first formant data set 5. The first formant data set 5 comprises formant frequencies associated with the letters in the word or words contained in the audio stream sound signal 3.

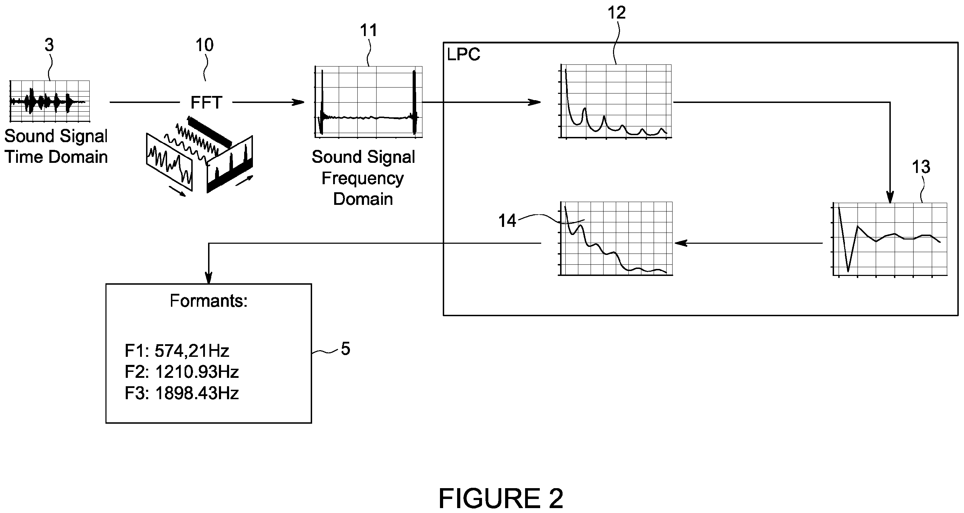

As shown in Figure 2, the steps for extracting the first formant data set 5 comprises converting the sound signal 3 from a time domain signal to a frequency domain signal 11, such as may be achieved by using a fast Fourier transform (FFT) algorithm 10, prior to application of various liner predictive coding (LPC) filters.

The liner predictive coding (LPC) steps involve, to the frequency domain signal 11, an autocorrelation algorithm 12 being applied to estimate the dominating frequency in the frequency domain signal 11. A Levinson-Durbin algorithm 13 is then applied to estimate linear prediction parameters for the estimated dominating frequency to compress the frequency domain signal and identify signal peaks in the compressed frequency domain signal.

The compressed frequency domain signal is then decompressed and the identified signal peaks from the decompressed frequency domain signal are extracted using a peak detector means at step 14.

The extracted signal peaks correspond to the formant frequencies in the first formant data set 5.

In the example illustrated in Figure 2, the extracted first formant data set 5 comprises formant frequencies F1 (Hz), F2 (Hz) and F3 (Hz) in which F1 = 574.21 Hertz, F2 = 1210.93 Hertz, and F3 = 1898.43 Hertz.

It will be however understood that although the extracted first formant data set 5 of Figure 2 shows formant frequencies F1 (Hz), F2 (Hz) and F3 (Hz), the extraction of formant frequencies F1 (Hz) and F2 (Hz) only may be used to determine the vowels spoken by an individual.

At step 6 of Figure 1, the vowels that have been spoken or articulated by the individual are determined by analysing the formant frequencies in the first formant data set 5.

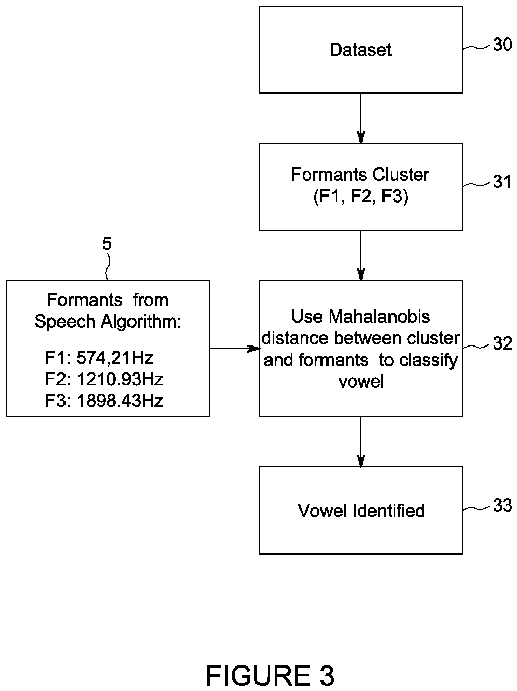

As shown in Figure 3, this stage comprises applying a Mahalanobis distance algorithm to the extracted formant frequencies at the first computing device to determine from the first formant data set formant frequencies that are associated with the one or more vowel letters and to determine the specific vowel letter.

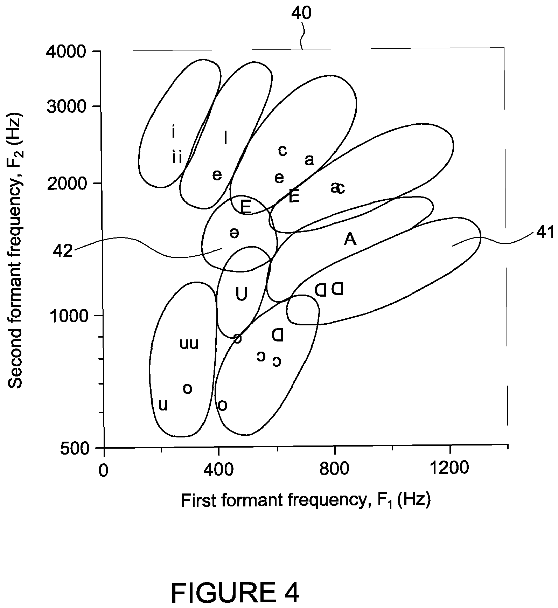

As shown in Figure 4, on a graph 40 of formant frequency F2 (Hz) against formant frequency F1 (Hz) data each vowel letter typically has frequencies that are clustered or distributed around a specific zone or area. For example, a cluster of frequencies for vowel letter "A" is indicated generally by the reference numeral 41, and a cluster of frequencies for vowel letter "E" is indicated generally by the reference numeral 42 etc. Using a distribution as shown in Figure 4, analysing what vowel letter has been spoken by an individual, comprises steps 30, 31 of classifying the formant frequencies 5 according to these known vowel frequency clusters or distributions 41, 42.

At step 32, a Mahalanobis distance algorithm is then used for measuring the distance between the extracted formant frequencies F1 (Hz) and F2 (Hz) and optionally F3 (Hz) and the cluster distribution of Figure 4, and output from the Mahalanobis distance algorithm will enable the identification of each vowel at step 33, together with the computation of mean formant frequency values for the individual for each vowel, for example, Vowel A - Mean values: F1: 800Hz, F2: 1450Hz and Vowel A - Mean values: F1: 875Hz, F2: 1490Hz as on for all other vowel letters.

Formant frequency data that are not associated with a vowel letter are discarded.

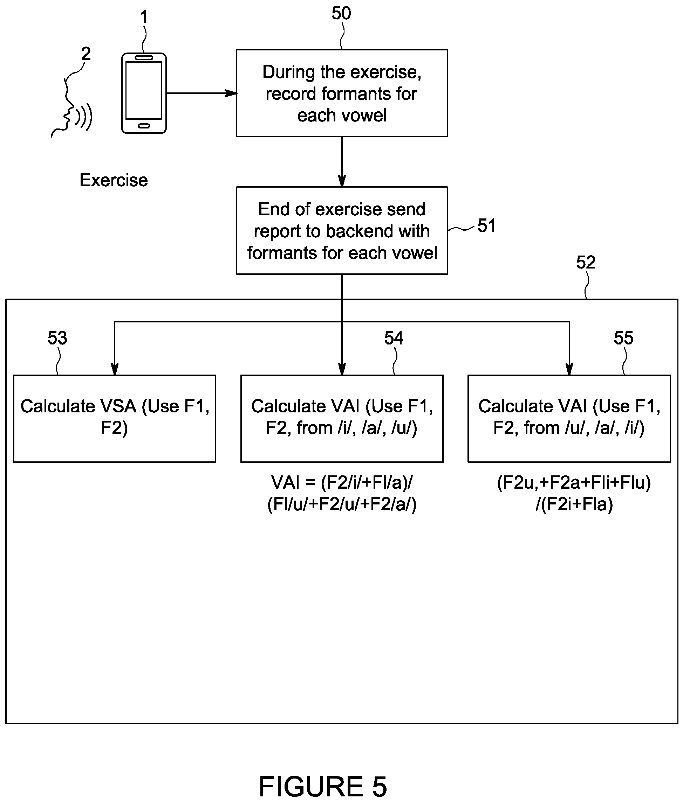

As shown in Figure 5, as the individual is performing an exercise in which they say or articulate a selection of words into a microphone of the first computing device, at step 50, a second formant data set comprising the mean formant frequencies for the specific vowel letter or vowel letters (as computed at step 33, Figure 3) for the individual are recorded.

At step 51 the recorded second formant data set 50 is recorded and comprises mean formant frequency values of F1 (Hz) and F2 (Hz) for the individual for each vowel.

A step of generating score data by applying one or more predetermined acoustic metrics to the recorded second formant data set 50 is then performed at the first computing device 1 (i.e. the individuals mobile phone), and/or at a second computing device 52 communicatively coupled to the first computing device 1 providing a backend computing capability.

As shown at step 7 of Figure 1, the predetermined metrics comprise one or more of: a formant centralisation ratio (FCR) algorithm, a vowel space algorithm (VSA) and a vowel articulation index (VAI) algorithm. In the instance shown in Figure 5, the backend second computing device 52 is applying a vowel space algorithm (VSA) 53 and vowel articulation index (VAI) algorithms 54, 55.

At step 8 in Figure 1, based on the score data generated, an assessment or inferences may be made (automatically or via manual clinician interpretation) to validate and check if the individual is saying the expected vowel. The score data if computed at the second computing device 52 may be transmitting as an output file to the first computing device 1 via a network to provide individuals with feedback.

A report on the level of articulation is generated using the score data which can allow for detection, diagnosis, prediction and/or monitoring progression of the condition, disorder, or disease.

It is to be understood that the invention is not limited to the specific details described herein which are given by way of example only and that various modifications and alterations are possible without departing from the scope of the invention.

## Claims

1. A system configured for analysing acoustic parameters of speech to detect, diagnose, predict and/or monitor progression of a condition, disorder, or disease, the system comprising a first computing device configured with means for receiving an audio stream containing speech data encoding at least one word spoken by an individual, the first computing device is configured with:
means to covert the audio stream into a sound signal,
means to extract from the sound signal a first formant data set comprising formant frequencies associated with letters in the at least one word as the audio stream is being received,
means to determine from the first formant data set formant frequencies that are associated with one or more vowel letters in the word,
means to determine the specific vowel letter or vowel letters from the determined vowel letter formant frequencies,
means for recording a second formant data set comprising at least some of the determined vowel letter formant frequencies for the specific vowel letter or vowel letters,
whereby the system further comprises, executing on the first computing device and/or a second computing device coupled to the first computing device by a network, means to generate score data by applying one or more predetermined acoustic metrics to the recorded second formant data set, in which the score data is used to determine a level of articulation of the vowel letter or vowel letters in the at least one word spoken by the individual, and
means to store the score data as an output file.

2. A system as claimed in Claim 1, in which the first computing device is a mobile computing device, such as a mobile smart phone, having mobile telephone and computing functionality.

3. A system as claimed in any one of the preceding claims, in which the means for extracting the first formant data set comprises:
speech analyser means configured with means for converting the sound signal from a time domain signal to a frequency domain signal, such as by using a fast Fourier transform (FFT) algorithm, and
linear predictive coding means comprising means for applying an autocorrelation algorithm to estimate the dominating frequency in the frequency domain signal, and means for applying a Levinson-Durbin algorithm to estimate linear prediction parameters for the estimated dominating frequency to compress the frequency domain signal and identify signal peaks in the compressed frequency domain signal, and
means for decompressing the compressed frequency domain signal, and
means for extracting the identified signal peaks from the decompressed frequency domain signal,
whereby the extracted signal peaks correspond to the formant frequencies in the first formant data set.

4. A system as claimed in any one of the preceding claims, in which the means to determine from the first formant data set formant frequencies that are associated with the one or more vowel letters and to determine the specific vowel letter comprises means for applying a Mahalanobis distance algorithm to the extracted formant frequencies at the first computing device.

5. A system as claimed in any one of the preceding claims, in which the predetermined metrics comprise one or more of: a formant centralisation ratio (FCR) algorithm, a vowel space algorithm (VSA) and a vowel articulation index (VAI) algorithm.

6. A system as claimed in any one of the preceding claims, in which the first computing device is a mobile phone.

7. A method configured for analysing acoustic parameters of speech to detect, diagnose, predict and/or monitor progression of a condition, disorder, or disease, the method comprising using a first computing device configured with means for receiving an audio stream containing speech data encoding at least one word spoken by an individual, the first computing device is configured to perform steps of:
converting the audio stream into a sound signal,
extracting from the sound signal a first formant data set comprising formant frequencies associated with the letters in the at least one word as the audio stream is being received,
determining from the first formant data set formant frequencies that are associated with one or more vowel letters in the word,
determining the specific vowel letter or vowel letters from the determined vowel letter formant frequencies,
recording a second formant data set comprising at least some of the determined vowel letter formant frequencies for the specific vowel letter or vowel letters,
whereby the method further comprises a step of, on the first computing device and/or a second computing device coupled to the first computing device by a network, generating score data by applying one or more predetermined acoustic metrics to the recorded second formant data set, in which the score data is used to determine a level of articulation of the vowel letter or vowel letters in the at least one word spoken by the individual, and
storing the score data as an output file.

8. The method as claimed in Claim 7, in which the first computing device is a mobile computing device, such as a mobile smart phone, having mobile telephone and computing functionality.

9. The method as claimed in any one of Claims 7 or 8, in which the step of extracting first formant data set comprises:
converting the sound signal from a time domain signal to a frequency domain signal, such as by using a fast Fourier transform (FFT) algorithm, and
applying an autocorrelation algorithm to estimate the dominating frequency in the frequency domain signal, and applying a Levinson-Durbin algorithm to estimate linear prediction parameters for the estimated dominating frequency to compress the frequency domain signal and identify signal peaks in the compressed frequency domain signal, and
decompressing the compressed frequency domain signal, and
extracting the identified signal peaks from the decompressed frequency domain signal,
whereby the extracted signal peaks correspond to the formant frequencies in the first formant data set.

10. The method as claimed in any one of Claims 7 to 9, in which the method comprises applying a Mahalanobis distance algorithm to the extracted formant frequencies at the first computing device to determine from the first formant data set formant frequencies that are associated with the one or more vowel letters and to determine the specific vowel letter.

11. The method as claimed in any one of Claims 7 to 10, in which the predetermined metrics comprise one or more of: a formant centralisation ratio (FCR) algorithm, a vowel space algorithm (VSA) and a vowel articulation index (VAI) algorithm.

12. The method as claimed in any one of Claims 7 to 11, in which the first computing device is a mobile phone.
